# EUROPEAN PATENT APPLICATION

(11) **EP 4 173 710 A1**
(43) Date of publication of application: **03.05.2023**
(21) Application number: 21306496.7
(22) Date of filing: 27.10.2021
(51) Int. Cl.: B01L 3/00, G01N 1/02, A61B 10/00

(54) **DEVICE FOR COLLECTING BIOLOGICAL SAMPLES**

(71) Applicant: BIC Violex Single Member S.A., 14569 Anoixi (GR); Société BIC, 92110 Clichy (FR)
(72) Inventor: Katsikas, Georgios, 145 69 Anoixi (GR); Saltas, Efthymios, 145 69 Anoixi (GR); Michenaud, Etienne, 92110 Clichy (FR)
(74) Representative: Peterreins Schley

(57) **Abstract**

The present disclosure relates to a device for collecting one or more biological samples comprising a housing and a collector element, wherein the collector element is configured to allow the passage of liquid, and wherein the collector element extends between a first end and a second end, the first end of the collector element protruding out of the housing for collecting the one or more biological samples, the second end of the collector element being arranged within the housing, and wherein the housing comprises a plurality of hooks for engaging the collector element.

## Description

### Background of the present disclosure

The present disclosure relates to device for collecting one or more biological samples, which may be subsequently used for molecular biology processing techniques such as nucleic acid amplification and/or detection.

Polymerase Chain Reaction (PCR) is a known method of amplifying nucleic acids, employed to test for the presence of specific nucleic acids (DNA or RNA) in a biological sample. It is used among other purposes as a diagnostic method to identify markers for pathogens or diseases. Other methods of amplifying nucleic acid samples include isothermal amplification methods such as Recombinase Polymerase Amplification (RPA) and Loop-Mediated Isothermal Amplification (LAMP).

Devices for collecting one or more biological samples - in particular for collecting saliva from the mouth of the consumer - are known from WO 2020/191232 A1, WO 2020/106345 A1, WO 2020/238035 A1 and EP 3 028 030 A1. Those devices comprise a wick as collector element. However, the wick is very soft and is difficult to maintain and fasten into the housing of the device.

The object of the present disclosure is to provide a device for collecting one or more biological samples having a collector element which can be mounted more easily on the device. In addition, the collector element should be securely mounted and maintained within the housing of the device to avoid an undesired removal.

### Summary of the present disclosure

The present disclosure is directed to a device for collecting one or more biological samples as defined in independent claim 1. The dependent claims depict embodiments of the present disclosure.

According to the present disclosure, a device for collecting one or more biological samples is provided which comprises a housing and a collector element, wherein the collector element is configured to allow the passage of liquid. It should be noted that the housing may comprise several separate housing parts. The collector element extends between a first end and a end, the first end of the collector element protruding out of the housing for collecting the one or more biological samples, the second end of the collector element being arranged within the housing. The first end may be arranged opposite with regard to the second end.

A plurality of hooks is provided for engaging the collector element. The number of hooks may be at least 8, specifically at least 20, most specifically at least 50. The height of the hooks may range from 0.5 mm to 3 mm, specifically from 0.8 mm to 2 mm (wherein the heigh is defined as the radial distance from the connection surface of the hooks to their maximum radial extension towards the axis of an opening in the housing in which the collector element is to be arranged, in the unbiased state of a hook).

According to an embodiment of the present disclosure, the front end of the housing may have an opening in which the collector element is arranged, wherein the hooks are formed on the inner surface of the opening. Alternatively, the hooks may be formed on a separate element which is held within the inner surface of the opening. This separate element may be manufactured like the hook part of a hook and loop fastener (also called "Velcro" fastener), for example by an injection molding process. If this hook part is manufactured as a planar part, it may be bent into a tubular shape and then glued into the opening of the housing.

The hooks are designed and arranged such that the forces needed for inserting the collector element into the housing are lower than the forces needed for removing the collector element out of the housing.

The hooks may generally have the shape of a "J" with the lower part of the "J" facing the axis of the opening. In particular, the tips of the hooks, i.e. the lower part of the "J", may face away from the front of the opening, i.e. toward the rear part of the housing which is opposite to the collector element. The hooks may be flexible so that the insertion of the collector element requires relatively low forces. On the other hand, the lower part of the "J" engages the surface of the collector element (and provides the function of a hook) so that the forces needed for removing the collector element are larger than the forces needed for inserting the collector element.

In other examples, the hooks may have the cross-sectional shape of a triangle, wherein a tip of the triangle faces the axis of the opening. The hooks in form of triangles may be stiff such that the collector element needs to be partially deformed when the collector element is inserted into the opening of the housing. The front surfaces of the triangles may be inclined having an angle ranging from 20 to 70 degrees with respect to the axis of the opening. With that, the front surfaces have a funneling effect when the collector element is inserted into the housing. The reverse surfaces of the triangles may have an angle ranging from 30 to 90 degrees with respect to the axis of the opening. Therefore, the triangles generally have the shape of a saw tooth or a shark fin so that the forces needed for removing the collector element are larger than the forces needed for inserting the collector element.

According to another embodiment of the present disclosure, the part of the collector element which is arranged within the housing is glued to the inner surface of the housing. As an example, a glue material - e.g. in form of a tubular sheet - may be arranged between the opening of the housing and the collector element, which can be activated by applying heat, ultraviolet light or microwaves. With that, the adhesive properties of the glue material may take place after the collector element has been introduced into the opening of the housing.

In all embodiments of the present disclosure, the following additional features may be provided.

The collector element has a certain softness so that the hooks can extend at least partially into the collector element when the second end of the collector element is mounted within the housing. The softness of the collector element may be such that, when mounted, a hook extends at least 0.1 mm into the collector element, specifically at least 0.2 mm, most specifically at least 0.3 mm, all compared to the undeformed state of the collector element. In addition or alternatively, the density of the collector element may be between 0.1 to 0.25 g/cm³.

The collector element may generally have an elongated shape extending from the first end to the second end of the collector element. In the following disclosure, the axis of the collector element is defined as being the central gravitational axis extending from the first end to the second end. The cross-sectional shape of the collector element (in a plane which is perpendicular to the axis of the collector element) may at least partially be circular, oval or rectangular (in examples, with rounded corners). The section of the collector element at its first end may have a conical section. In addition or alternatively, the first end of the collector element may be pointed.

The purpose of the collector element is to collect and/or absorb one or more biological samples. In case of dry biological samples, the biological samples may stick on the outer surface of the first end of the collector element. In case of biological samples which are contained in a liquid, the biological samples may be absorbed within the collector element. Therefore, the collector element should allow the passage of liquid at least partially in order to take up absorb a greater amount of the biological sample(s). The material of the collector element may be porous or may have an internal channel structure, and may have hydrophilic properties in order to absorb a large amount of liquid biological samples more quickly.

Suitable materials for the collector element include cotton-based materials, or a plastic based matrix such as PE (polyethylene), PP (polypropylene) or PVDF (polyvinylidene fluoride) or a combination thereof, which can be manufactured in a range of densities to retain biological samples of different viscosities. In examples, the collector element may be made of a combination of PE and PP. The PP may be the core portion and the PE may be the outer coating, due to its particular properties on absorption of liquid via its capillary structure, and adsorption which is minimal with regards to nucleic acids. The collector element may comprise fibers (e.g. pressed fibers) made of one or more of these materials. The material of the collector element may be porous or may have an internal channel structure, and may have hydrophilic properties in order to absorb a large amount of liquid biological samples more quickly. The porosity may be at least 50%, more specifically at least 70%, most specifically at least 80%.

In addition, as will be explained below in more detail, the collector element may allow the passage of liquid from a container, in particular a buffer solution and/or a reagent solution, through the collector element towards its first end in order to drain the biological sample(s) out of the collector element. In particular, the collector element may allow the passage of liquid from its second end to its first end, or from the outer surface near the second end to the first end.

As already mentioned, the collector element may be soft and/or flexible such that the anchor element can extend at least partially into the collector element. In addition, the device may comprise an abutment surface or a stop for limiting the movement of the collector element into the housing.

The device may further comprise a container for storing liquid, in particular a buffer solution and/or a reagent solution therein. In addition or alternatively, the housing may comprise or enclose a channel for guiding the liquid stored in the container to the collector element. The device may further comprise a release mechanism for opening a passage from the container to the channel so that liquid stored in the container can enter the channel from the container and reach the collector element. This release mechanism may, for example, be a valve or a cover sheet on one side of the container which can be penetrated by a needle by moving the container further into the housing.

Therefore, liquid stored in the container can reach the collector element through the channel when the release mechanism is open, wherein at least part of the liquid can pass through the collector element by gravity acting on the liquid when the container is in a position vertically above the collector element. In other examples, the volume of the container can be reduced after the release mechanism has opened the fluid passage from the container, for example by a movable plunger or by providing the container with flexible walls such that the liquid can be squeezed out of the container into the channel.

When the liquid from the container passes through the collector element, the biological sample can at least partially be washed out of the collector element for testing/analysis purposes.

The device may further comprise a cap element which can be fitted on the housing on the side on which the collector element is arranged. An element for indicating and/or measuring a reaction between a reagent and the at least one biological sample may be arranged within or on the cap element, in particular an element indicating the pH value of the mixture of the liquid from the container and of the at least one biological sample. In addition or alternatively, the cap element may comprises a reagent or an element comprising a reagent which is released to the mixture of liquid from the container and of the at least one biological sample. With these features, the device can be used not only for collecting biological samples, but also to analyze/test the samples on site (i.e. without the need of sending the device to a laboratory). And finally, the cap element may comprise a window which is arranged such that a user can at least partially see the element for indicating and/or measuring a reaction between the reagent and the at least one biological sample.

### Short description of the figures

- Figure 1: shows a device for collecting one or more biological samples according to an embodiment of the present disclosure;
- Figures 2a to 2c: show the anchoring of a collector element according to a first embodiment of the present disclosure;
- Figure 3: show the anchoring of a collector element according to a second embodiment of the present disclosure;
- Figures 4a and 4b: show the connection of a collector element according to a third embodiment of the present disclosure; and
- Figure 5: show a cross-sectional drawing of a device according to the present disclosure.

### Detailed description of the present disclosure

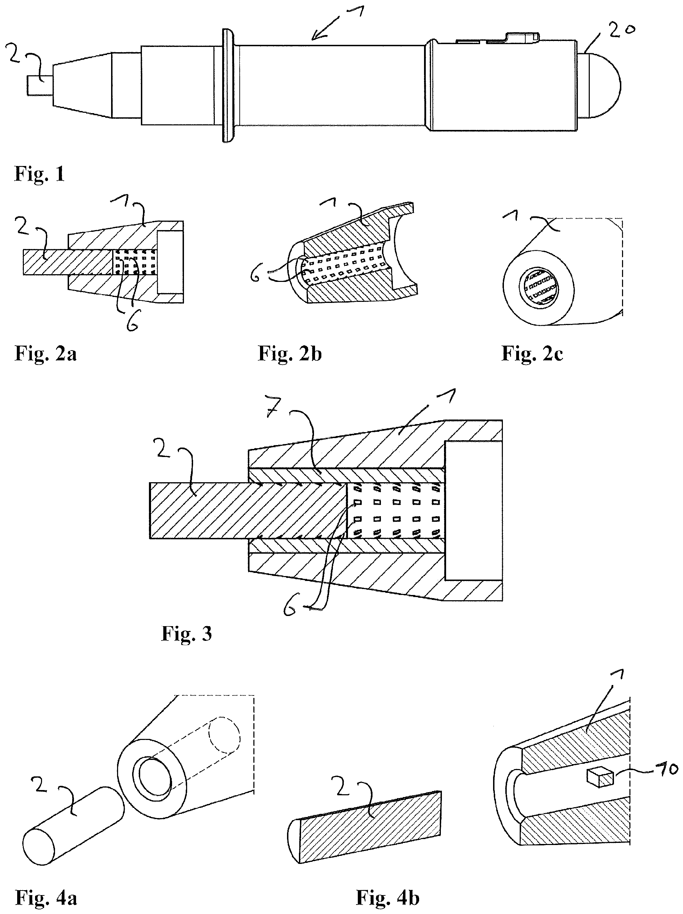

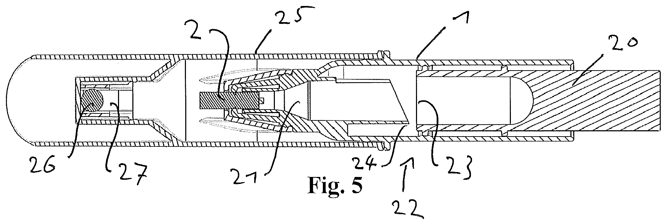

Figure 1 shows a device for collecting one or more biological samples according to an embodiment of the present disclosure. The device comprises a housing 1 and a collector element 2, wherein the collector element 2 allows the passage of liquid. As already mentioned, the housing may consist of several separate housing parts. The collector element 2 has two ends, the first end of the collector element protruding out of the housing 1 for collecting the one or more biological samples, the second end of the collector element 2 being arranged within the housing 1. Regarding the softness range, the shape, the absorption properties and the material of the collector element 2, it is referred to the above summary section of the present disclosure. The device may also comprise a container 20 for storing liquid, in particular a buffer solution and/or a reagent solution therein as will be explained in more detail in context with Figure 5.

Figures 2a to 2c and Figure 3 show the anchoring of a collector element 2 according to a first and second embodiment of the present disclosure for maintaining the position of the collector element with respect to the housing 1. According to these embodiments, a plurality of hooks 6 is provided for engaging the collector element. The number of hooks may be at least 8, specifically at least 20, most specifically at least 50. The height of the hooks 6 may range from 0.5 mm to 3 mm, specifically from 0.8 mm to 2 mm (wherein the heigh is defined as the radial distance from the connection surface of the hooks to their maximum radial extension towards the axis of an opening in the housing in which the collector element is to be arranged, in the unbiased state of a hook).

The front end of the housing 1 has an opening in which the collector element 2 is arranged. In the embodiment shown in Figures 2a and 2b, the hooks 6 are formed on the inner surface of the opening. The front end of the housing which includes the opening may be manufactured as an integral part of the housing 1. Alternatively, this front part may be manufactured separately and then mounted on the main body of the housing 1.

The collector element 2 may simply be mounted into the opening of the housing by pushing the collector element 2 in axial direction into the opening.

In the embodiment shown in Figure 3, the hooks are formed on a separate element 7 which is held within the inner surface of the opening. This separate element 7 may be manufactured like the hook part of a hook and loop fastener (also called "Velcro" fastener), for example by an injection molding process. If this hook part is manufactured as a planar part, it may be bent into a tubular shape and then glued into the opening of the housing.

In all embodiments, the hooks 6 are designed and arranged such that the forces needed for inserting the collector element into the housing are lower than the forces needed for removing the collector element out of the housing.

The hooks 6 may generally have the shape of a "J" with the lower part of the "J" facing the axis of the opening. In particular, the tips of the hooks, i.e. the lower part of the "J", may face away from the front of the opening, i.e. toward the rear part of the housing which is opposite to the collector element. The hooks may be flexible so that the insertion of the collector element requires relatively low forces. On the other hand, the lower part of the "J" engages the surface of the collector element (and provides the function of a hook) so that the forces needed for removing the collector element are larger than the forces needed for inserting the collector element.

In other examples, the hooks 6 may have the cross-sectional shape of a triangle (not shown in the Figures), wherein a tip of the triangle faces the axis of the opening. The hooks in form of triangles may be stiff such that the collector element needs to be partially deformed when the collector element is inserted into the opening of the housing. The front surfaces of the triangles may be inclined having an angle ranging from 20 to 70 degrees with respect to the axis of the opening. With that, the front surfaces have a funneling effect when the collector element is inserted into the housing. The reverse surfaces of the triangles may have an angle ranging from 30 to 90 degrees with respect to the axis of the opening. Therefore, the triangles generally have the shape of a saw tooth or a shark fin so that the forces needed for removing the collector element are larger than the forces needed for inserting the collector element.

Figures 4a and 4b show the connection of a collector element according to a third embodiment of the present disclosure. According to this embodiment, the part of the collector element 2 which is arranged within the housing is glued to the inner surface of the housing. As an example, a glue material - e.g. in form of a tubular sheet - may be arranged between the opening of the housing and the collector element, which can be activated by applying heat, ultraviolet light or microwaves. With that, the adhesive properties of the glue material may take place after the collector element has been introduced into the opening of the housing.

In all embodiments of the present disclosure, the device may comprise an abutment surface or a stop 10 for limiting the movement of the collector element 2 into the housing.

In all embodiments of the present disclosure, the collector element may have a softness as described above in the summary section such that the anchor element can extend at least partially into the collector element.

Suitable materials for the collector element include cotton-based materials, or a plastic based matrix such as PE, PP or PVDF which can be manufactured in a range of densities to absorb and retain biological samples of different viscosities. The material of the collector element may be porous or may have an internal channel structure, and may have hydrophilic properties in order to absorb a large amount of liquid biological samples more quickly. The porosity may be at least 50%, more specifically at least 70%, most specifically at least 80%.

Figure 5 show a cross-sectional drawing of a device according to the present disclosure in which the anchoring of the collector element 2 may be designed according to one of the embodiments described in this disclosure (wherein Figure 5 shows an alternative anchoring of the collector element). The device may comprise a container 20 for storing liquid, in particular a buffer solution. In addition, the housing may comprise or enclose a channel 21 for guiding the liquid stored in the container 20 to the collector element 2. The device may further comprise a release mechanism 22 for opening a passage from the container 20 to the channel 21 so that liquid stored in the container can enter the channel 21 from the container 20 and reach the collector element 2. This release mechanism may, for example, be a valve or a cover sheet 23 on one side of the container which can be penetrated by a needle or by a penetrating device 24 by moving the container further into the housing.

Therefore, liquid stored in the container can reach the collector element 2 through the channel 21 when the release mechanism 22 is open, wherein at least part of the liquid can pass through the collector element 2 by gravity acting on the liquid when the container is in a position vertically above the collector element. In other examples, the volume of the container 20 be designed to be reduced after the release mechanism has opened the fluid passage from the container, for example by a movable plunger or by providing the container 20 with flexible walls such that the liquid can be squeezed out of the container into the channel. When the liquid from the container 20 passes through the collector element 2, the biological sample can at least partially be washed out of the collector element 2 for testing/analysis purposes.

The device may further comprise a cap element 25 which can be fitted on the housing on the side on which the collector element is arranged. The cap element may comprise a reagent 26 or an element comprising a reagent which is released to the mixture of liquid from the container and of the at least one biological sample. This element comprising a reagent may have the shape of a pellet. In addition, an element 27 for indicating and/or measuring a reaction between a reagent 26 and the at least one biological sample may be arranged in a reaction chamber of the cap element, in particular an element indicating the pH value of the mixture of the liquid from the container and of the at least one biological sample. With these features, the device can be used not only for collecting biological samples, but also to analyze/test the samples on site (i.e. without the need of sending the device to a laboratory).

The cap element 25 may comprise a window (not shown in the Figures) which is arranged such that a user can at least partially see the element for indicating and/or measuring a reaction between the reagent and the at least one biological sample. In other examples, the cap element may be manufactured of a transparent material.

According to this disclosure, various amplification methods may be used, for example a recombinase polymerase amplification (RPA) which is a low temperature DNA and/or RNA amplification technique, or a ligase chain reaction (LCR). The buffer liquid stored in the container 20 may contain purified water, milli-q water and buffer (e.g. TRIS-Cl / TE buffer), for example a Tris-HCl buffer. When LCR method is applied, buffer also contains PEG as crowding agent.

The reagents located in the cap may include a group of materials, such as primers, probes additionally to the enzymes (ligase or polymerase) and nucleotides. Generally, the reagents located in the cap are dependent on the used amplification method. A suitable reagent composition for RPA may contain enzymes (in particular enzymes for polymerase and/or enzymes for reverse transcriptase), proteins, primers, probes, oligonucleotides and reaction components necessary for the RPA reaction including reaction buffer, polyethylene glycol, pyrophosphatase, a regeneration system such as kinase/phosphocreatine system, or a mixture thereof. A suitable reagent composition for LCR may contain enzymes for DNA ligase and/or DNA probes.

Possible materials for the collector element have already been described. The collector element may additionally be treated. For example, reagents or active agents may be covalently bound to the collector element by chemical linkages through functionalisation (e.g. -OH groups), or they may be dried into the material of the collector element and become active on hydration with the sample. Alternatively or in addition, the collector element may be treated to provide means of lysing virus, in particular a treatment to form a negatively charged surface (so as to retain the positively charged ions of the sample) or a treatment with anionic detergent. Possible active agents that render the collector element functionalized may lyse bacteria or viruses. These active agents may be quaternary ammonium compounds (QAC) and/or biocidal agents. Examples of these active agents are: Citral, Eucalyptus oil, Tea tree oil, Chlorhexidine, triterpenoid saponin, Polyphylla saponin I, Povidone-iodine, Cetyl pyridinium chloride (CPC), Benzalkonium chloride (BAC), Dequoalinium chloride.

## Claims

1. A device for collecting one or more biological samples comprising a housing and a collector element,
wherein the collector element is configured to allow the passage of liquid, and
wherein the collector element extends between a first end and a second end,
the first end of the collector element protruding out of the housing for collecting the one or more biological samples,
the second end of the collector element being arranged within the housing,
wherein the housing comprises a plurality of hooks for engaging the collector element.

2. Device according to claim 1, wherein the front end of the housing has an opening in which the collector element is arranged, wherein the hooks are formed on an inner surface of the opening, or wherein the hooks are formed on a separate element which is held within an inner surface of the opening.

3. Device according to claim 1 or 2, wherein the hooks are designed such that the forces needed for inserting the collector element into the housing are lower than the forces needed for removing the collector element out of the housing.

4. A device for collecting one or more biological samples comprising a housing and a collector element,
wherein the collector element allows the passage of liquid, and
wherein the collector element extends between a first end and a second end,
the first end of the collector element protruding out of the housing for collecting the one or more biological samples,
the second end of the collector element being arranged within the housing,
wherein the part of the collector element which is arranged within the housing is glued to the inner surface of the housing.

5. Device according to one of the preceding claims, wherein the collector element is soft and/or flexible such that the anchor element can extend at least partially into the collector element.

6. Device according to one of the preceding claims, further comprising an abutment surface or a stop for limiting the movement of the collector element into the housing.

7. Device according to one of the preceding claims, further comprising a container for storing liquid, in particular a buffer solution and/or a reagent solution therein.

8. Device according to claim 7, wherein the housing comprises or encloses a channel for guiding the liquid stored in the container to the collector element.

9. Device according to one of the preceding claims, further comprising a valve and/or release mechanism for opening a passage from the container to the channel so that liquid stored in the container can enter the channel from the container and reach the collector element.

10. Device according to claim 9, wherein liquid stored in the container can reach the collector element through the channel when the valve and/or release mechanism is open, wherein at least part of the liquid can pass through the collector element by gravity acting on the liquid when the container is in a position vertically above the collector element.

11. Device according to one of claims 7 to 10, wherein the volume of the container can be reduced to squeeze liquid from the container into the channel.

12. Device according to one of claims 7 to 11, wherein the volume of the container can be reduced such that liquid is released from the container into the channel and passes through the collector element.

13. Device according to one of the preceding claims, further comprising a cap element which can be fitted on the housing on the side on which the collector element is arranged.

14. Device according to one of the preceding claims, wherein an element for indicating and/or measuring a reaction between a reagent and the at least one biological sample is arranged within or on the cap element, in particular an element indicating the pH value of the mixture of the liquid from the container and of the at least one biological sample.

15. Device according to one of the preceding claims, wherein the cap element comprises a reagent or an element comprising a reagent which is released to the mixture of liquid from the container and of the at least one biological sample, wherein the cap element preferably comprises a window which is arranged such that a user can at least partially see the element for indicating and/or measuring a reaction between the reagent and the at least one biological sample.
